# EUROPÄISCHE PATENTSCHRIFT

(11) **EP 1 185 206 B1**
(45) Veröffentlichungstag und Bekanntmachung des Hinweises auf die Patenterteilung: **20.08.2003**
(21) Anmeldenummer: 00940354.4
(22) Anmeldetag: 09.06.2000
(51) Int. Cl.: A61B 17/28, A61B 17/02

(54) **MEDIZINISCHES GREIFINSTRUMENT**
MEDICAL GRIPPING INSTRUMENT
PINCE MEDICALE

(30) Priorität: 11.06.1999 DE 19926555
(43) Veröffentlichungstag der Anmeldung: 13.03.2002
(73) Patentinhaber: Karl Storz GmbH & Co. KG, 78532 Tuttlingen (DE)
(72) Erfinder: WÖLLMER, Wolfgang, D-20257 Hamburg (DE)
(74) Vertreter: Hofmeister, Frank Horst
(86) Internationale Anmeldenummer: EP0005342
(87) Internationale Veröffentlichungsnummer: WO00076407

(56) Entgegenhaltungen:
- US-A- 5 201 759
- US-A- 5 318 013
- US-A- 5 456 695

## Beschreibung

Die Erfindung betrifft ein medizinisches Greifinstrument, insbesondere zur Verwendung in der Mikrolaryngoskopie, mit einem langgestreckten Schaft, an dessen distalem Ende ein zwischen einer geschlossenen und einer geöffneten Stellung verstellbares Zangenteil und an dessen proximalem Ende eine aus einem starren Griffteil und einem gegenüber dem starren Griffteil verschwenkbaren Griffteil bestehende Handhabe angeordnet sind, wobei das Ver stellen des Zangenteils über eine in dem Schaft gelagerte, mit dem verschwenkbaren Griffteil verbundene Schub-/ Zugstange erfolgt, sowie mit einem vom Schaft abwinkelbaren Spreizteil.

Aus der mikrochirurgischen und insbesondere laserchirurgischen Mikrolaryngoskopie ist es bekannt bei Operationen, bei denen Gewebe entfernt werden soll, durch den Schaft eines starren Endoskops ein Greifinstrument in den Körper des Patienten einzuführen, um einerseits das Abtrennen des Gewebeteils mittels eines weiteren medizinischen Instruments, beispielsweise eines Lasers, zu erleichtern und um andererseits das abgetrennte Gewebe aus dem Körper zu entfernen. Insbesondere bei laserchirurgischen Eingriffen besteht die Gefahr, daß zu schonendes Gewebe in Mitleidenschaft gezogen wird, namentlich durch die thermische Belastung, die mit der Verwendung des Lasers verbunden ist.

Ein gattungsgemäßes medizinisches Greifinstrument ist beispielsweise aus der US 5,456,695 A bekannt. Dieses bekannte Greifinstrument weist eine aus zwei Griffteilen bestehende Handhabe auf, über die ein Zangenteil am distalen Ende des Schaftes geöffnet und geschlossen werden kann. Zusätzlich zu diesem Zangenteil am distalen Ende des Schaftes ist in einem mittleren Bereich des Schaftes ein fächerförmiges Spreizteil angeordnet, über welches einzelne als Retraktoren dienende Fächerblätter in der Verstellebene des Zangenteils vom Schaft abwinkelbar sind, um beispielsweise Gewebe zur Seite zu drücken. Das Verstellen des Spreizteils aus der im Schaft liegenden Ruhestellung in die abgewinkelte Spreizstellung erfolgt durch Drehen eines Ringes, der am distalen Ende der Handhabe, also im Übergangsbereich zum Schaft angeordnet ist.

Neben dem Umstand, daß es bei diesem bekannten Greifinstrument notwendig ist, immer mit beiden Händen zu arbeiten, wenn man gleichzeitig die Zangenteile und das Spreizteil betätigen will, da das Betätigen der Griffteile und das Verdrehen des Ringes nicht mit einer Hand zu bewerkstelligen sind, ist es bei dieser Vorrichtung nachteilig, daß das Spreizteil zum proximalen Ende des Schaftes hin verschoben von den Zangenteilen beabstandet ist und außerdem in der Verstellebene der Zangenteile arbeitet. Durch diese Ausgestaltung des Spreizteils ist es insbesondere bei beengten Platzverhältnissen am Operationsgebiet, wie dies beispielsweise bei der Laryngoskopie der Fall ist, kaum möglich, nicht zu behandelndes Gewebe so zu schützen, daß nur das von den Zangenteilen ergriffene Gewebe dem operativen Eingriff ausgesetzt ist.

Ausgehend von diesem Stand der Technik liegt der Erfindung die **Aufgabe** zugrunde, ein medizinisches Greifinstrument der eingangs genannten Art zu schaffen, welches einfach aufgebaut und leicht zu handhaben ist und darüber hinaus gewährleistet, daß nicht zu entfernendes Gewebe geschont wird.

Die **Lösung** dieser Aufgabenstellung ist dadurch gekennzeichnet, daß das vom Schaft abwinkelbare Spreizteil zusätzlich zu dem Zangenteil am distalen Ende des Schaftes angeordnet und in einer Ebene in etwa rechtwinklig zur Verstellebene des Zangenteils vom Schaft abwinkelbar ist.

Durch die Anordnung des Spreizteils am distalen Ende des Schaftes, also in unmittelbarer Nähe der Zangenteile, ist mit dem erfindungsgemäßen Greifinstrument möglich, auch bei beengten Platzverhältnissen am Operationsgebiet zu erhaltendes Gewebe in unmittelbar an den mit den Zangenteilen ergriffenen Operationsbereich angrenzenden Bereichen so zur Seite zu drängen, daß diese bei dem operativen Eingriff nicht in Mitleidenschaft gezogen werden. Die Abwinklung des Spreizteils in etwa rechtwinklig zur Verstellebene der Zangenteile ermöglicht weiterhin eine weitestgehend unabhängige Bewegung des Spreizteils einerseits und der Zangenteile andererseits.

Die Handhabung des erfindungsgemäßen Greifinstruments und insbesondere die einhändige Bedienbarkeit wird dadurch ermöglicht, daß das Abwinkeln des Spreizteils über ein zusätzliches Griffteil der Handhabe erfolgt.

Durch die Verwendung dieses mit dem zusätzlichen Spreizteil versehenen Greifinstruments ist es möglich, zu erhaltendes Gewebe gezielt aus dem Operationsgebiet herauszuhalten, um Platz und Sicht für den chirurgischen Eingriff zu schaffen. Vorteilhaft an dem erfindungsgemäßen Greifinstrument ist darüber hinaus, daß es direkt über die Handhabe des Greifinstruments betätigbar ist und dieses zusätzliche Hilfsmittel somit dem Operateur jederzeit zur Verfügung steht.

Das Bedienen dieses erfindungsgemäß ausgebildeten medizinischen Instruments kann dadurch erleichtert werden, daß das verschwenkbare Griffteil zum Verstellen des Zangenteils in seiner jeweiligen Stellung gegenüber dem starren Griffteil feststellbar ist. Durch das Feststellen des Zangenteils in der Greifstellung hat der Operateur jetzt die Möglichkeit, das Spreizteil völlig unabhängig von der Betätigung des Zangenteils zu benutzen.

Zum Feststellen des Zangenteils dient gemäß einer bevorzugten Ausführungsform der Erfindung ein an einem der beiden Griffteile angeordneter Rastarm, der mit einem an dem anderen Griffteil angeordneten Rastelement zusammenwirkt. Der Rastarm weist dabei vorteilhafterweise eine sägezahnartig profilierte Oberfläche auf.

Gemäß einer praktischen Ausführungsform der Erfindung wird vorgeschlagen, daß der Rastarm verschwenkbar an einem der Griffteile gelagert ist und in Richtung auf die Raststellung federbelastet ist.

Eine besonders gute Handhabbarkeit des erfindungsgemäßen Greifinstruments ergibt sich, wenn sowohl das Zangenteil als auch das Spreizteil um bis zu 90°, vorzugsweise 50°, zu öffnen bzw. vom Schaft abwinkelbar sind.

Weitere Merkmale und Vorteile der Erfindung ergeben sich anhand der nachfolgenden Beschreibung der zugehörigen Zeichnung, in der ein Ausführungsbeispiel eines erfindungsgemäßen medizinischen Greifinstruments dargestellt ist. In der Zeichnung zeigt:
- Fig. 1a: eine Seitenansicht eines erfindungsgemäßen medizinischen Greifinstruments mit geschlossenem Zangenteil und angelegtem Spreizteil;
- Fig.1b: eine Draufsicht auf die Darstellung gemäß Fig. 1a;
- Fig. 2a: eine Seitenansicht des medizinischen Greifinstruments gemäß Fig.1a, jedoch mit geöffnetem Zangenteil und abgewinkeltem Spreizteil und
- Fig.2b: eine Draufsicht auf die Darstellung gemäß Fig. 2a.

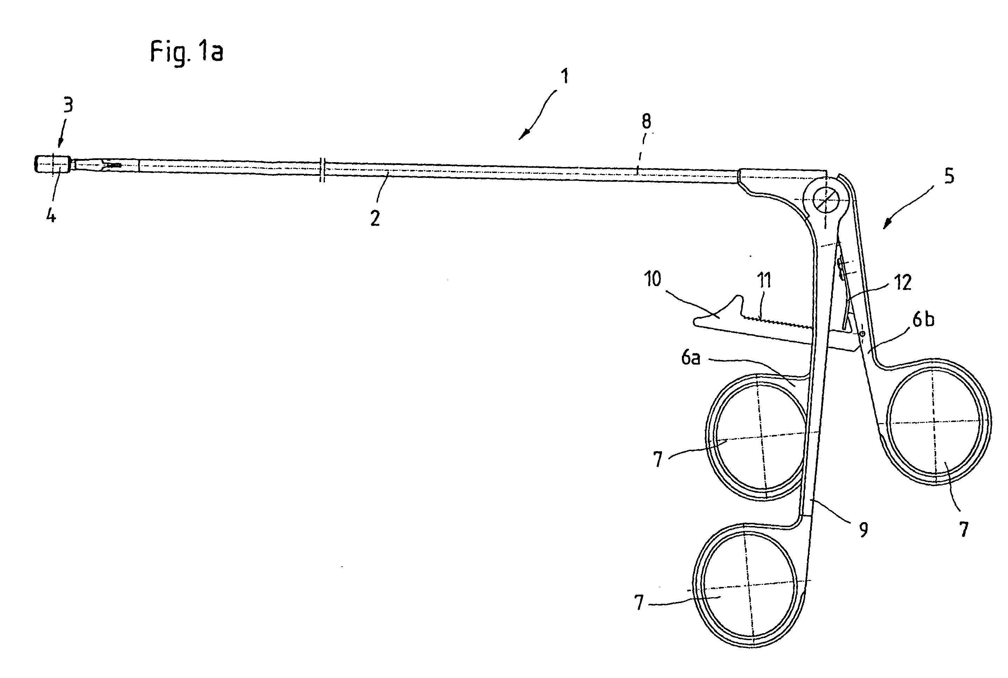

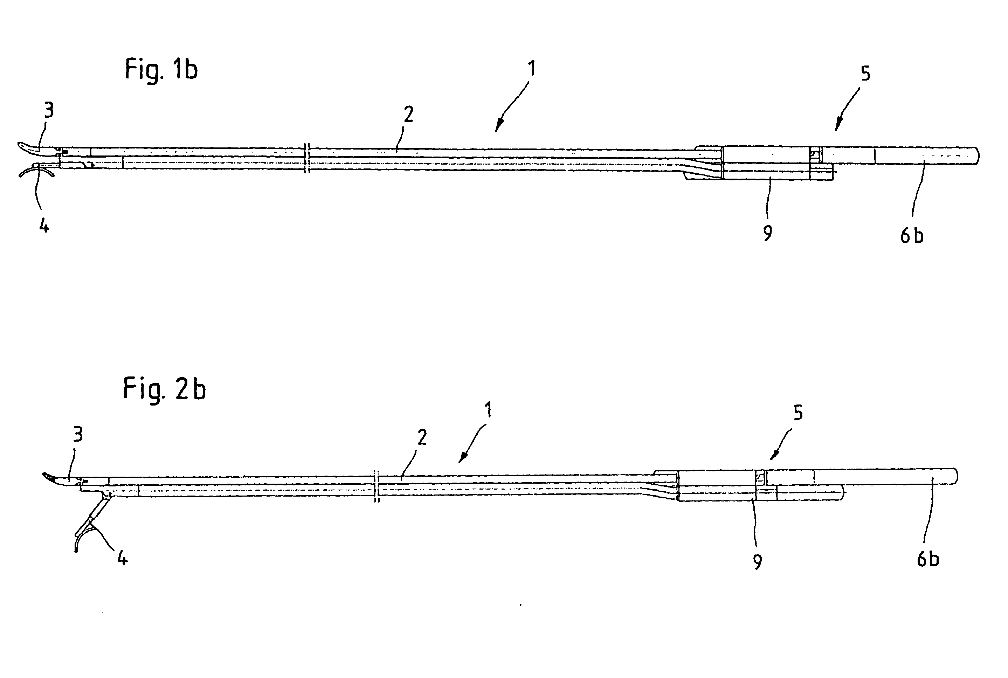

Fig. 1a zeigt ein als Faßzange 1 ausgebildetes medizinisches Greifinstrument, das insbesondere zur Verwendung bei mikrolaryngoskopischen Eingriffen geeignet ist. Die Faßzange 1 weist einen langgestreckten Schaft 2 auf, an dessen distalem Ende ein Zangenteil 3 sowie ein vom Schaft 2 der Faßzange 1 abwinkelbares Spreizteil 4 angeordnet sind, wie dies insbesondere den Abbildungen Fig. 1b und Fig. 2b zu entnehmen ist.

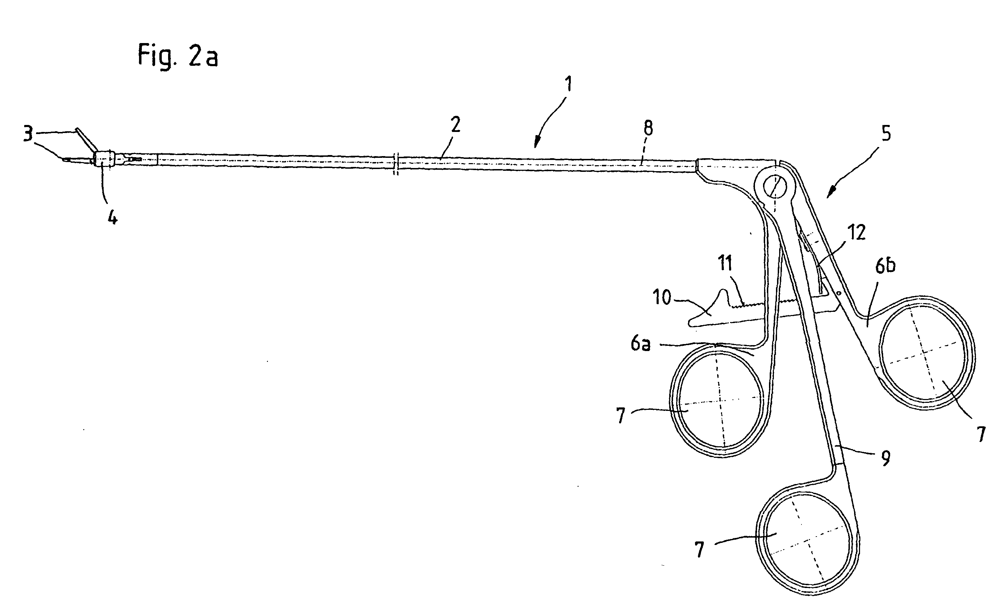

Am proximalen Ende des Schaftes 2 ist eine Handhabe 5 angeordnet, über die sowohl das Zangenteil 3 als auch das Spreizteil 4 betätigbar sind. Zum Betätigen des Zangenteils 3 dienen das starre Griffteil 6a sowie das gegenüber dem starren Griffteil 6a verschwenkbare Griffteil 6b, welche jeweils mit Fingerösen 7 zur Steuern der Griffteile 6a und 6b versehen sind. Das Verstellen des Zangenteils 3 zwischen der in Fig.1a und 1b dargestellten geschlossenen Position und der in Fig. 2a und 2b dargestellten geöffneten Position erfolgt über eine in dem Schaft 2 der Faßzange 1 gelagerte Schub-/ Zugstange 8, die an einem Ende mit dem Zangenteil 3 und am anderen Ende mit dem verschwenkbaren Griffteil 6b verbunden ist.

Das Betätigen des ebenfalls am distalen Ende der Faßzange 1 angeordneten Spreizteils 4 erfolgt bei dem dargestellten Ausführungsbeispiel über ein zusätzliches verschwenkbares Griffteil 9 der Handhabe 5. Beim dargestellten Ausführungsbeispiel eines medizinischen Greifinstruments ist das ebenfalls mit einer Fingeröse 7 versehene Griffteil 9 des Spreizteils 4 länger ausgebildet als die Griffteile 6a und 6b zum Betätigen des Zangenteils 3. Die Länge der einzelnen Griffteile 6a, 6b und 9 hat jedoch keine Auswirkung auf die Funktionsweise eines solchermaßen ausgebildeten medizinischen Greifinstruments.

Wie weiterhin aus Fig. 1a und Fig. 2a ersichtlich ist, ist am verschwenkbaren Griffteil 6b zum Betätigen des Zangenteils 3 ein Rastarm 10 angeordnet, der an seiner Oberseite mit einer Sägezahnprofilierung 11 versehen ist, die mit einem nicht dargestellten, am starren Griffteil 6a angeordneten Rastelement so zusammenwirkt, daß die Griffteile 6a, 6b zur Betätigung des Zangenteils 3 und somit das Zangenteil 3 selbst in seiner jeweiligen Position feststellbar ist.

Das Arbeiten mit dem dargestellten Greifinstrument geschieht wie folgt:

Die Faßzange 1 wird in der in Fig.1a dargestellten Stellung durch den Schaft eines nicht dargestellten Endoskops in den Körper des Patienten eingeführt. Anschließend wird durch Betätigen der Griffteile 6a und 6b das Zangenteil 3 geöffnet und das zu entfernende Gewebe mit dem Zangenteil 3 ergriffen. Über den mit der Sägezahnprofilierung 11 versehenen Rastarm 10 läßt sich das verschwenkbare Griffteil 6b durch Zusammenwirken mit dem am starren Griffteil 6a angeordneten Rastelement in der gegenüber dem starren Griffteil 6a verschwenkten Stellung feststellen. Das Zangenteil 3 hält nunmehr ohne weitere Betätigung durch den Operateur das zu entfernende Gewebe fest gegriffen.

Um Platz und freie Sicht für den chirurgischen Eingriff zu schaffen, kann der Operateur jetzt über das verschwenkbare Griffteil 9 das Spreizteil 4 vom Schaft 2 der Faßzange 1 abwinkeln und zu schonendes Gewebe aus dem Operationsgebiet herausdrücken. Insbesondere bei laserchirurgischen Eingriffen ist die Verwendung des Spreizteils 4 vorteilhaft, da so das zu schonende Gewebe vor der thermischen Belastung durch den Laser geschützt werden kann.

Während des chirurgischen Eingriffs wird das zu entfernende Gewebe über die Faßzange 1 mit Zug belastet, wodurch ein schnelles und sauberes Abtrennen des Gewebes gewährleistet wird.

Nach dem chirurgischen Eingriff wird das Spreizteil wieder an den Schaft 2 der Faßzange 1 angelegt und die Faßzange 1 mitsamt dem über das Zangenteil 3 ergriffenen Gewebe wieder aus dem Körper des Patienten herausgezogen.

Das Lösen der Griffteile 6a, 6b aus der verrasteten Stellung erfolgt dadurch, daß der verschwenkbar am Griffteil 6b gelagerte Rastarm 10 entgegen der Kraft einer den Rastarm 10 in die Raststellung belastenden Feder 12 abwärts gedrückt wird, bis das Rastelement außer Eingriff mit der Sägezahnprofilierung 11 des Rastarms 10 tritt.

Mit einem solchermaßen ausgebildeten medizinischen Greifinstrument ist es für den Operateur nunmehr erstmalig möglich, bei mikrolaryngoskopischen Eingriffen mit nur einem Instrument zu schonendes Gewebe gezielt aus dem Operationsgebiet herauszuhalten.

### Bezugszeichenliste

- 1: Faßzange
- 2: Schaft
- 3: Zangenteil
- 4: Spreizteil
- 5: Handhabe
- 6a: starres Griffteil
- 6b: verschwenkbares Griffteil
- 7: Fingeröse
- 8: Schub-/ Zugstange
- 9: zusätzliches Griffteil
- 10: Rastarm
- 11: Sägezahnprofilierung
- 12: Feder

## Patentansprüche

1. Medizinisches Greifinstrument, insbesondere zur Verwendung in der Mikrolaryngoskopie, mit einem langgestreckten Schaft (2), an dessen distalem Ende ein zwischen einer geschlossenen und einer geöffneten Stellung verstellbares Zangenteil (3) und an dessen proximalem Ende eine aus einem starren Griffteil (6a) und einem gegenüber dem starren Griffteil (6a) verschwenkbaren Griffteil (6b) bestehende Handhabe (5) angeordnet sind, wobei das Verstellen des Zangenteils (3) über eine in dem Schaft (2) gelagerte, mit dem verschwenkbaren Griffteil (6b) verbundene Schub-/ Zugstange (8) erfolgt, sowie mit einem vom Schaft (2) abwinkelbaren Spreizteil (4),
**dadurch gekennzeichnet,**
**daß** das vom Schaft (2) abwinkelbare Spreizteil (4) zusätzlich zu dem Zangenteil (3) am distalen Ende des Schaftes (2) angeordnet und in einer Ebene in etwa rechtwinklig zur Verstellebene des Zangenteils (3) vom Schaft (2) abwinkelbar ist.

2. Medizinisches Greifinstrument nach Anspruch 1, **dadurch gekennzeichnet, daß** das Spreizteil (4) über ein zusätzliches verschwenkbares Griffteil (9) betätigbar ist.

3. Medizinisches Greifinstrument nach Anspruch 1 oder 2, **dadurch gekennzeichnet, daß** das verstellbare Griffteil (6b) zum Verstellen des Zangenteils (3) in seiner jeweiligen Stellung gegenüber dem starren Griffteil (6a) feststellbar ist.

4. Medizinisches Greifinstrument nach Anspruch 3, **dadurch gekennzeichnet, daß** zum Feststellen der zum Verstellen des Zangenteils (3) dienenden Griffteile (6a, 6b) an einem (6b) der beiden Griffteile ein Rastarm (10) angeordnet ist, der mit einem am anderen Griffteil (6a) angeordneten Rastelement zusammenwirkt.

5. Medizinisches Greifinstrument nach Anspruch 4, **dadurch gekennzeichnet, daß** der Rastarm (10) eine sägezahnförmig profilierte Oberfläche aufweist.

6. Medizinisches Greifinstrument nach Anspruch 4 oder 5, **dadurch gekennzeichnet, daß** der Rastarm (10) verschwenkbar an einem der Griffteile (6b) gelagert ist und in Richtung auf die Raststellung federbelastet ist.

7. Medizinisches Greifinstrument nach mindestens einem der Ansprüche 1 bis 6, **dadurch gekennzeichnet, daß** das Zangenteil (3) um bis zu 90°, vorzugsweise 50°, zu öffnen ist.

8. Medizinisches Greifinstrument nach mindestens einem der Ansprüche 1 bis 7, **dadurch gekennzeichnet, daß** das Spreizteil (4) um bis zu 90°, vorzugsweise 50°, vom Schaft (2) abwinkelbar ist.

## Claims

1. Medical gripping instrument, especially for use in microlaryngoscopy, with an elongate shaft (2) at whose distal end a pincer part (3) is arranged which can be adjusted between a closed position and an opened position, and at whose proximal end a handle (5) is arranged which consists of a rigid grip part (6a) and of a grip part (6b) which can be pivoted in relation to the rigid grip part (6a), the pincer part (3) being adjusted via a push/pull rod (8) which is mounted in the shaft (2) and is connected to the pivotable grip part (6b), and also with a spreading part (4) which can be angled away from the shaft (2), **characterized in that** the spreading part (4) which can be angled away from the shaft (2) is arranged additionally to the pincer part (3) at the distal end of the shaft (2) and can be angled away from the shaft (2) in a plane approximately perpendicular to the plane of adjustment of the pincer part (3).

2. Medical gripping instrument according to Claim 1, **characterized in that** the spreading part (4) can be actuated via an additional pivotable grip part (9).

3. Medical gripping instrument according to Claim 1 or 2, **characterized in that** the adjustable grip part (6b) for adjusting the pincer part (3) can be fixed in its respective position in relation to the rigid grip part (6a).

4. Medical gripping instrument according to Claim 3, **characterized in that**, in order to fix the grip parts (6a, 6b) used for adjusting the pincer part (3), a locking arm (10) is arranged on one (6b) of the two grip parts, said locking arm (10) cooperating with a locking element arranged on the other grip part (6a).

5. Medical gripping instrument according to Claim 4, **characterized in that** the locking arm (10) has a surface with a serrated profile.

6. Medical gripping instrument according to Claim 4 or 5, **characterized in that** the locking arm (10) is mounted pivotably on one of the grip parts (6b) and is spring-loaded in the direction of the locked position.

7. Medical gripping instrument according to at least one of Claims 1 to 6, **characterized in that** the pincer part (3) can be opened by up to 90°, preferably by 50°.

8. Medical gripping instrument according to at least one of Claims 1 to 7, **characterized in that** the spreading part (4) can be angled away from the shaft (2) by up to 90°, preferably by 50°.

## Revendications

1. Instrument médical de préhension, en particulier pour l'utilisation dans la micro-laryngoscopie, comportant un tronc allongé (2) à l'extrémité distale duquel est agencée une partie de pince (3) déplaçable entre une position fermée et une position ouverte, et à l'extrémité proximale duquel est agencée une manette (5) constituée par une partie de poignée rigide (6a) et par une partie de poignée (6b) basculante par rapport à la partie de poignée rigide (6a), le déplacement de la partie de pince (3) s'effectuant via une barre poussée/tirée (8) montée dans le tronc (2) et reliée à la partie de poignée basculante (6b), et comportant une partie d'écartement (4) susceptible d'être coudée depuis le tronc (2), **caractérisé en ce que** la partie d'écartement (4) susceptible d'être coudée depuis le tronc (2) est agencée en supplément à la partie de pince (3) à l'extrémité distale du tronc (2) et est susceptible d'être coudée depuis le tronc (2) dans un plan approximativement à angle droit par rapport au plan de déplacement de la partie de pince (3).

2. Instrument médical de préhension selon la revendication 1, **caractérisé en ce que** la partie d'écartement (4) peut être actionnée via une partie de poignée basculante supplémentaire (9).

3. Instrument médical de préhension selon l'une ou l'autre des revendications 1 et 2, **caractérisé en ce que** la partie de poignée déplaçable (6b) est susceptible d'être immobilisée dans sa position respective par rapport à la partie de poignée rigide (6a) pour déplacer la partie de pince (3).

4. Instrument médical de préhension selon la revendication 3, **caractérisé en ce que** pour immobiliser les parties de poignée (6a, 6b) servant à déplacer la partie de pince (3), un bras d'enclenchement (10) est agencé sur l'une (6b) des deux parties de poignée, qui coopère avec un élément d'enclenchement agencé sur l'autre partie de poignée (6a).

5. Instrument médical de préhension selon la revendication 4, **caractérisé en ce que** le bras d'enclenchement (10) présente une surface profilée en forme de dents de scie.

6. Instrument médical de préhension selon l'une ou l'autre des revendications 4 et 5, **caractérisé en ce que** le bras d'enclenchement (10) est monté mobile en basculement sur l'une des parties de poignée (6b) et est chargé par un ressort en direction de la position d'enclenchement.

7. Instrument médical de préhension selon l'une des revendications 1 à 6, **caractérisée en ce que** la partie de pince (3) peut être ouverte jusqu'à 90°, de préférence 50°.

8. Instrument médical de préhension selon l'une des revendications 1 à 7, **caractérisé en ce que** la partie d'écartement (4) est susceptible d'être coudée depuis le tronc (2) jusqu'à 90°, de préférence 50°.
